(19) Europäisches Patentamt
European Patent Office
Office européen des brevets

(11) Veröffentlichungsnummer: **0 191 724**
**A2**

(12) # EUROPÄISCHE PATENTANMELDUNG

(21) Anmeldenummer: **86810005.8**

(22) Anmeldetag: **10.01.86**

(51) Int. Cl.⁴: **C 07 D 413/14**, C 07 D 413/04, A 61 K 31/44

(30) Priorität: **15.01.85 CH 167/85**

(43) Veröffentlichungstag der Anmeldung: **20.08.86 Patentblatt 86/34**

(84) Benannte Vertragsstaaten: **AT BE CH DE FR GB IT LI LU NL SE**

(71) Anmelder: **SANDOZ AG, Lichtstrasse 35, CH-4002 Basel (CH)**
(84) Benannte Vertragsstaaten: **BE CH FR GB IT LI LU NL SE**

(71) Anmelder: **SANDOZ-PATENT-GMBH, Humboldtstrasse 3, D-7850 Lörrach (DE)**
(84) Benannte Vertragsstaaten: **DE**

(71) Anmelder: **SANDOZ-ERFINDUNGEN Verwaltungsgesellschaft m.b.H., Brunner Strasse 59, A-1235 Wien (AT)**
(84) Benannte Vertragsstaaten: **AT**

(72) Erfinder: **Berthold, Richard, Ahornstrasse 9, CH-4103 Bottmingen (CH)**
Erfinder: **Vogel, Arnold, Hellring 9, CH-4125 Riehen (CH)**

(54) **1,4-Dihydropyridine, Verfahren zu ihrer Herstellung und diese enthaltende pharmazeutische Zusammensetzungen.**

(57) 4-Aryl-1,4-dihydropyridine-3- and/or 5-carboxylic acid ester derivatives wherein at least one ester group is additionally substituted by a β-blocker moiety, in particular of formula I

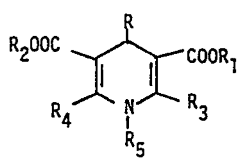

I

wherein the substituents have various significances, with the proviso that they are other than compounds of formula Y

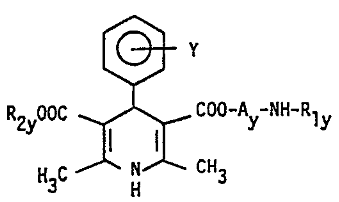

Y

wherein the substituents have various significances, have calcium-antagonistic and β-adrenoceptor blocking activity. They may be used as dualistic calcium antagonists and β-adrenoceptor blocking agents.

EP 0 191 724 A2

0191724

SANDOZ AG.
Basle

Case 100-6545

1,4-DIHYDROPYRIDINE DERIVATIVES, THEIR PREPARATION AND PHARMACEUTICAL COMPOSITIONS CONTAINING THEM

The present invention relates to 1,4-dihydropyridine derivatives, their preparation and pharmaceutical compositions containing them.

In particular the invention provides a 4-aryl-1,4-dihydropyridine-3-and/or 5- carboxylic acid ester derivative wherein at least one ester group is additionally substituted by a β-blocker moiety,

with the proviso that it is other than a compound of formula Y

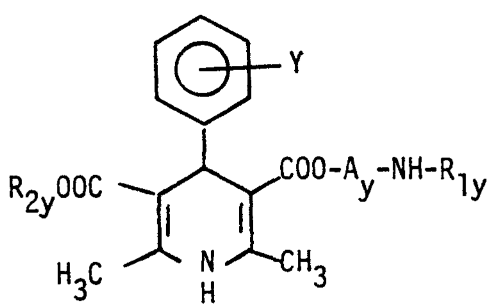

Y

wherein

either

Y is nitro,

$R_{1y}$ is 2-hydroxy-2-phenylethyl or 2-hydroxy-3-phenoxypropyl,

$R_{2y}$ is alkyl of 1 to 10 carbon atoms and

$A_y$ is alkylene of 1 to 10 carbon atoms optionally interrupted by an oxygen atom

or

Y is hydrogen, nitro or trifluoromethyl,

$R_{1y}$ is 2-hydroxy-3-phenoxypropyl optionally substituted in the phenyl ring by halogen, lower alkyl, allyl, lower alkoxy(lower)-alkyl, allyloxy, cyano or ethinyl, or is 2-hydroxy-3-indolyl-oxypropyl,

$R_{2y}$ is lower alkyl and

$A_y$ is $-CH_2CR^yR^{y'}-$ wherein $R^y$ and $R^{y'}$ independently are hydrogen or methyl.

This ester derivative is hereinafter referred to as "a compound of the invention". It preferably is a 3,5-dicarboxylic acid ester derivative.

In a subgroup $A_y$ is alkylene of 1 to 10 carbon atoms optionally interrupted by one or more oxygen atoms or a group $-CH_2CR^yR^{y'}-$ as defined above.

"Aryl" as used herein includes heteroaryl. It optionally is substituted. It preferably is heteroaryl.

A β-blocker moiety is a moiety characterized by the structure

$-\underset{|}{N}CH_2\underset{\underset{O-}{|}}{C}H-aryl-$, $-\underset{|}{N}CH_2\underset{\underset{O-}{|}}{C}HCH_2O-aryl-$, $-aryl-\underset{\underset{O-}{|}}{C}HCH_2\underset{|}{N}-$

or $-aryl-OCH_2\underset{\underset{O-}{|}}{C}HCH_2\underset{|}{N}-$ . In a subgroup it is characterized by the structure $-\underset{|}{N}CH_2\underset{\underset{O-}{|}}{C}HCH_2O-aryl-$ or $-aryl-OCH_2\underset{\underset{O-}{|}}{C}HCH_2N-$ .

The ester group may be substituted by the β-blocker moiety directly or over a substituent on the aryl part of the β-blocker moiety, such as oxygen or carbamoyl. When the amino group of the β-blocker moiety is terminal it preferably is substituted, preferably monosubstituted. The oxy group of the β-blocker moiety preferably is unsubstituted, i.e. it preferably is hydroxy. It may however also be substituted, e.g. esterified. When it is esterified it e.g. is a group $-OCOR_e$ wherein

$R_e$ is alkyl of 1 to 12 carbon atoms, cycloalkyl of 3 to 7 carbon atoms, phenyl, phenylalkyl of 7 to 12 carbon atoms, phenyl or phenylalkyl of 7 to 12 carbon atoms monosubstituted in the phenyl ring by alkyl of 1 to 4 carbon atoms, phenyl or phenylalkyl of 7 to 12 carbon atoms monosubstituted or independently disubstituted in the phenyl ring by halogen of atomic number of from 9 to 35, or phenyl or phenylalkyl of 7 to 12 carbon atoms mono- or independently di- or independently trisubstituted in the phenyl ring by alkoxy of 1 to 4 carbon atoms.

The amino and oxy parts in a β-blocker moiety may be in cyclized form, e.g. as an oxazolidine, e.g. as a 2-phenyloxazolidine. They preferably are in free, open form.

Preferably only one ester group is substituted by a β-blocker moiety.

In a further subgroup the derivative defined above is other than a compound of formula Y'

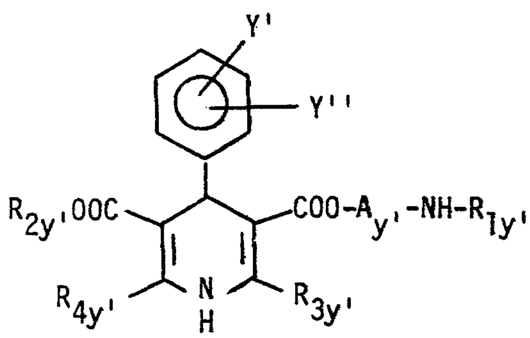

Y'

wherein

either

$R_{1y'}$ is 2-hydroxy-2-phenylethyl; 2-hydroxy-3-phenoxypropyl optionally substituted in the phenyl ring by cyano, halogen of atomic number of from 9 to 53, alkoxy of 1 to 4 carbon atoms or alkanoyl of altogether 1 to 5 carbon atoms; or 2-hydroxy-3-(naphth-1-yl)oxypropyl,

$R_{2y'}$ is alkyl of 1 to 10 carbon atoms optionally interrupted by one or more oxygen atoms, or is $-(CH_2)_{ny}NR_yR_{y'}$ wherein ny is 1 or 2 and $R_y$ and $R_{y'}$ independently are alkyl of 1 to 4 carbon atoms or phenylalkyl of 7 to 10 carbon atoms,

$R_{3y'}$ and $R_{4y'}$ independently are alkyl of 1 to 4 carbon atoms,

$A_{y'}$ is alkylene of 1 to 10 carbon atoms optionally interrupted by one or more oxygen atoms and

Y' and Y'' independently are hydrogen, nitro, halogen of atomic number of from 9 to 53, trifluoromethyl, cyano, alkyl of 1 to 4 carbon atoms, alkoxy of 1 to 4 carbon atoms or alkenyloxy of 2 to 4 carbon atoms,

or

$R_{1y'}$ is 2-hydroxy-3-phenoxypropyl optionally substituted in the phenyl ring by halogen, lower alkyl, allyl, loweralkoxy-(lower)alkyl, allyloxy, cyano or ethinyl, or is 2-hydroxy-3-indolyloxypropyl,

$R_{2y'}$ is lower alkyl,

$R_{3y'}$ and $R_{4y'}$ are methyl,

$A_{y'}$ is $-CH_2CR^yR^{y'}-$ wherein $R^y$ and $R^{y'}$ independently are hydrogen or methyl,

Y' is hydrogen and

Y'' is hydrogen, nitro or trifluoromethyl.

The invention thus provides e.g. a compound of formula I

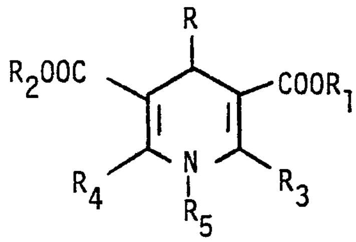

I

wherein

R is optionally substituted aryl,

$R_1$ is a group usual in the 4-aryl-1,4-dihydropyridine-3,5-dicarboxylic acid ester field but additionally substituted by a β-blocker moiety,

$R_2$ is a group usual in the 4-aryl-1,4-dihydropyridine-3,5-dicarboxylic acid ester field or independently has the significance indicated above for $R_1$ and

$R_3$, $R_4$ and $R_5$ independently are a group usual in the 4-aryl-1,4-dihydropyridine-3,5-dicarboxylic acid ester field,

with the proviso that it is other than a compound of formula Y as defined above.

In a subgroup it is other than a compound of formula Y' as defined above.

R is e.g. carbocyclic aryl such as phenyl optionally substituted by nitro, preferably monosubstituted; or optionally substituted by halogen of atomic number of from 9 to 35, alkyl of 1 to 4 carbon atoms, alkoxy of 1 to 4 carbon atoms, or cyano, preferably monosubstituted; or is a heterocyclic group, e.g. as indicated hereunder for $R^a$.

A preferred group of compounds of formula I is the compounds of formula I'

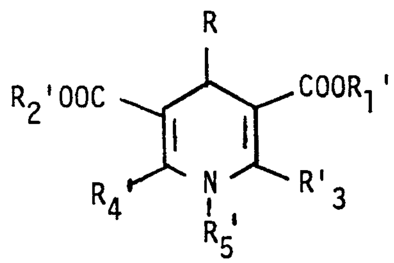

I'

wherein

R is as defined above,

$R_1$' is a hydrocarbyl group which is substituted by an optionally substituted 3-amino-2-oxypropoxyaryl, 3-amino-2-oxypropoxy-aryloxy or 3-aryloxy-2-oxypropylamino moiety, is optionally interrupted by 1 oxygen atom and/or 1-NH- or -N-alkyl-group and may be further substituted, whereby the carbonyloxy part in formula I' is separated from any oxygen or nitrogen atom by at least 2 carbon atoms,

$R_2$' has the significance indicated above for $R_1$' or is a hydro-carbyl group which is optionally interrupted by 1 oxygen atom and/or 1-NH- or -N-alkyl-group and may be further substituted,

$R_3$' and $R_4$' independently are hydrogen, alkyl, aryl, aralkyl, cyano, hydroxymethyl, methoxymethyl, amino, aminomethyl or (2-amino)ethoxymethyl and

$R_5$' is hydrogen or optionally substituted alkyl,

with the proviso that it is other than a compound of formula Y as defined above.

In a subgroup it is other than a compound of formula Y' as defined above. A 3-amino-2-oxypropoxyaryl, 3-amino-2-oxypropoxy-aryloxy or 3-aryloxy-2-oxypropylamino moiety may be bound over a substituent in the aryl part thereof, e.g. a carbamoyl or oxy group, to the hydrocarbyl moiety.

A particularly preferred group of compounds of formula I is the compounds of formula Ia

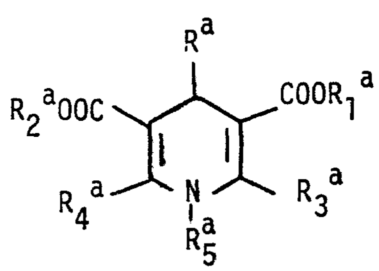

Ia

wherein

$R^a$ is a carbocyclic aryl group or thienyl, furyl, pyrryl, pyrazolyl, imidazolyl, oxazolyl, isoxazolyl, thiazolyl, pyridyl, pyridazinyl, pyrimidyl, pyrazinyl, indolyl, benzimidazolyl, benzoxazolyl, benzisoxazolyl, benzoxadiazolyl, benzothiadiazolyl, quinolyl, isoquinolyl, quinazolyl or quinoxalyl whereby the carbocyclic aryl group and the heterocyclic groups optionally are mono- or independently disubstituted by phenyl, alkyl, alkoxy, alkylen, dioxy-alkylen, halogen, trifluoromethyl, polyfluoroalkoxy, hydroxy, nitro, cyano, azido, alkylthio, alkylsulfinyl or alkylsulfonyl,

$R_1^a$ is a hydrocarbyl group which is substituted by an optionally substituted 3-amino-2-oxypropoxyaryl, 3-amino-2-oxypropoxy-aryloxy or 3-aryloxy-2-oxypropylamino moiety, is optionally interrupted by 1 oxygen atom and may be further substituted, whereby the carbonyloxy part in formula Ia is separated from any oxygen or nitrogen atom by at least 2 carbon atoms,

$R_2^a$ has the significance indicated above for $R_1^a$ or is a hydrocarbyl group which is optionally interrupted by 1 oxygen atom and may be further substituted,

$R_3^a$ and $R_4^a$ independently are hydrogen, alkyl, aryl, aralkyl or cyano and

$R_5^a$ is hydrogen or optionally substituted alkyl,

with the proviso that it is other than a compound of formula Y as defined above.

In a sub group it is other than a compound of formula Y' as defined above.

R and $R^a$ preferably are phenyl substituted by nitro, cyano or chlorine, especially nitro, 2,1,3-benzoxadiazolyl unsubstituted or substituted by chlorine ,or pyridyl optionally substituted by cyano.

$R_1$' and $R_1^a$ preferably are a hydrocarbyl group substituted by an optionally substituted 3-amino-2-oxypropoxyaryloxy or 3-amino-2-oxypropoxyaryl moiety.

$R_2$' and $R_2^a$ preferably are a hydrocarbyl group.

$R_3$', $R_3^a$, $R_4$' and/or $R_4^a$ preferably are hydrogen, alkyl , amino or cyano. Preferably they are alkyl.

$R_5$' and $R_5^a$ preferably are hydrogen.

A hydrocarbyl group preferably is alkyl of 1 or 2 to 16 carbon atoms, preferably of 1 or 2 to 14 carbon atoms, especially of 1 to 3 carbon atoms. When it is not substituted by a 3-amino-2-oxypropoxyaryl, 3-amino-2-oxypropoxyaryloxy or 3-aryloxy-2-oxypropylamino moiety, it preferably is of 1 to 6, especially 1 to 4 carbon

atoms , especially methyl or isopropyl. When it is bound to such a propyl moiety it preferably is of 7 to 14, especially of 9 to 12, especially of 10 carbon atoms and preferably is alkylene, preferably unbranched. A hydrocarbyl group preferably is either unsubstituted or substituted by such a propyl moiety only.

An even more preferred group of compoundsof formula I is the compounds of formula Iaa

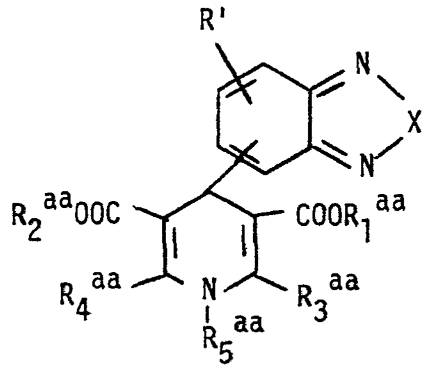

Iaa

wherein

X is an oxygen or sulfur atom,

R' is hydrogen, alkyl of 1 to 4 carbon atoms, alkoxy of 1 to 4 carbon atoms, alkylthio of 1 to 4 carbon atoms, alkylsulfonyl of 1 to 4 carbon atoms, halogen of atomic number of from 9 to 35, trifluoromethyl, nitro or hydroxy,

$R_1^{aa}$ is an alkylene group of at least 2 carbon atoms which is substituted by an optionally substituted 3-amino-2-oxypropoxyaryl, 3-amino-2-oxy-propoxyaryloxy or 3-aryloxy-2-oxypropylamino moiety, whereby the carbonyloxy part in formula Iaa is separated from any oxygen or nitrogen atom by at least 2 carbon atoms,

$R_2^{aa}$ has the significance indicated above for $R_1^{aa}$ or is alkyl of 1 to 12 carbon atoms; hydroxyalkyl of 2 to 12 carbon atoms wherein the hydroxy part is separated from the carbonyloxy moiety by at least 2 carbon atoms; alkoxyalkyl of 3 to 12 carbon atoms wherein the alkoxy part is separated from the carbonyloxy moiety by at least 2 carbon atoms; alkenyl of 3 to 6 carbon atoms wherein the double bond is separated from the carbonyloxy moiety by at least 1 carbon atom not participating in the double bond; alkinyl of 3 to 6 carbon atoms wherein the triple bond is separated from the carbonyloxy moiety by at least 1 carbon atom not participating in the triple bond; cycloalkyl of 3 to 7 carbon atoms; cycloalkylalkyl of 4 to 8 carbon atoms; hydroxyalkoxyalkyl of 4 to 8 carbon atoms wherein the hydroxyalkoxy part is separated from the carbonyloxy moiety by at least 2 carbon atoms and the hydroxy part is separated from the oxygen atom of the alkoxy part by at least 2 carbon atoms; phenyl or phenylalkyl of 7 to 10 carbon atoms optionally mono- or independently disubstituted in the phenyl ring by alkyl of 1 to 4 carbon atoms, alkoxy of 1 to 4 carbon atoms or halogen of atomic number of from 9 to 35; or aminoalkyl of 2 to 6 carbon atoms wherein the nitrogen atom is separated from the carbonyloxy moiety by at least 2 carbon atoms and the amino part is optionally mono- or independently disubstituted by alkyl of 1 to 4 carbon atoms, phenyl, phenylalkyl of 7 to 10 carbon atoms, or phenyl or phenylalkyl of 7 to 10 carbon atoms themselves mono- or independently disubstituted in the phenyl ring by alkyl of 1 to 4 carbon atoms, alkoxy of 1 to 4 carbon atoms or halogen of atomic number of from 9 to 35,

$R_3^{aa}$ and $R_4^{aa}$ independently are hydrogen, alkyl of 1 to 6 carbon atoms or cyano and

$R_5^{aa}$ is hydrogen; alkyl of 1 to 6 carbon atoms; alkenyl of 3 to 6 carbon atoms wherein the double bond is separated from the nitrogen atom by at least 1 carbon atom not participating in the double bond; alkinyl of 3 to 6 carbon atoms wherein the triple bond is separated from the nitrogen atom by at least 1 carbon atom not participating in the triple bond; cycloalkyl of 3 to 7 carbon atoms; cycloalkylalkyl of 4 to 8 carbon atoms; hydroxyalkyl of 2 to 6 carbon atoms wherein the hydroxy part is separated from the nitrogen atom by at least 2 carbon atoms; alkoxyalkyl of 2 to 6 carbon atoms; or phenylalkyl of 7 to 9 carbon atoms or phenylalkenyl of 9 to 12 carbon atoms wherein the double bond is separated from the nitrogen atom by at least 1 carbon atom not participating in the double bond, the last two substituents optionally being independently mono-, di- or trisubstituted in the phenyl ring by independently alkyl of 1 to 4 carbon atoms, alkoxy of 1 to 4 carbon atoms, halogen of atomic number of from 9 to 35 or hydroxy.

X preferably is oxygen. R' preferably is hydrogen or halogen, especially chlorine. The 1,4-dihydropyridinyl group is preferably bound in formula Iaa to the 4 position on the heterocycle. When R' is other than hydrogen then the 1,4-dihydropyridinyl group preferably is bound to the 5 or 7 position.

$R_5^{aa}$ preferably is hydrogen. When it is other than hydrogen it preferably is alkyl, cycloalkylalkyl, optionally substituted phenylalkyl or alkoxyalkyl, preferably alkyl or alkoxyalkyl, especially alkyl.

Preferably one of $R_3^{aa}$ and $R_4^{aa}$ is alkyl and the other is cyano or alkyl, especially alkyl.

$R_1^{aa}$ and $R_2^{aa}$ preferably are different. One of $R_1^{aa}$ or $R_2^{aa}$ preferably is alkyl.

When $R_5^{aa}$ is alkyl it preferably is branched at the carbon atom adjacent to the nitrogen atom. When $R_5^{aa}$ is alkoxyalkyl then the alkoxy part thereof preferably is separated from the nitrogen atom of the 1,4-dihydropyridinyl moiety by at least 2 carbon atoms.

Alkyl of 1 to 12 carbon atoms preferably is of 1 to 6 carbon atoms, especially of 1 to 4 carbon atoms, it particularly is methyl, ethyl or isopropyl. Alkyl of 1 to 6 carbon atoms preferably is of 1 to 4, especially 1 to 3 carbon atoms, it particularly is methyl or isopropyl. When alkyl of 1 to 4 carbon atoms is a phenyl ring or amino substituent it preferably is of 1 or 2 carbon atoms, it especially is methyl. When alkoxy of 1 to 4 carbon atoms is a phenyl ring substituent it preferably is of 1 or 2 carbon atoms, it especially is methoxy. When halogen of atomic number of from 9 to 35 is a phenyl ring substituent it preferably is chlorine or bromine, especially chlorine.

Alkenyl and/or alkinyl preferably are of 3 carbon atoms. Cycloalkyl and/or the cycloalkyl part of cycloalkylalkyl preferably are of 3, 5 or 6 carbon atoms, especially of 5 or 6 carbon atoms.

The alkylene part of cycloalkylalkyl and/or of phenylalkyl of 7 to 9 carbon atoms or of 7 to 10 carbon atoms preferably is of 1 or 2 carbon atoms, it especially is methylene.

The alkenylene part of phenylalkenyl preferably is of 3 carbon atoms.

The alkylene part of hydroxyalkyl of 2 to 6 carbon atoms and/or of alkoxyalkyl of 2 to 6 carbon atoms and/or of hydroxyalkoxyalkyl and/or of optionally substituted aminoalkyl preferably is of 2 or 3 carbon atoms, especially of 2 carbon atoms.

The alkylene part of hydroxyalkyl of 2 to 12 carbon atoms preferably is of 2 to 6 carbon atoms, especially of 2 or 3 carbon atoms, particularly of 2 carbon atoms. Alkoxyalkyl of 3 to 12 carbon atoms and/or of 2 to 6 carbon atoms preferably is of 3 to 6, especially of 3 carbon atoms.

The hydroxy part of hydroxyalkyl and/or of hydroxyalkoxyalkyl and/or the amino part of aminoalkyl preferably is attached to the distal, terminal carbon atom. Hydroxyalkyl of 2 to 12 carbon atoms preferably is of 2 to 6, especially of 2 carbon atoms. The alkoxy part of hydroxyalkoxyalkyl preferably is of 2 carbon atoms. The alkoxy part of alkoxyalkyl preferably is of 1 or 2 carbon atoms, it especially is methoxy.

Where a phenyl ring is present in a substituent other than as the aryl part of a β-blocker moiety, it is preferably unsubstituted. When it is substituted it preferably is monosubstituted, preferably in the para position. When it is disubstituted it preferably is substituted in the ortho and para positions. When it is trisubstituted it preferably is substituted in the meta, meta and para positions. Alkoxy and/or halogen are preferred as substituents on such a phenyl ring substituent. When such a phenyl ring is polysubstituted the substituents preferably are identical.

The amino part of aminoalkyl preferably is substituted, especially disubstituted. Preferred substituents of the amino part of aminoalkyl are alkyl and/or optionally substituted phenylalkyl. Preferably the amino part of aminoalkyl is disubstituted by alkyl and optionally substituted phenylalkyl.

The hydrocarbyl part of $R_1'$ and $R_1^a$ and the alkylene part of $R_1^{aa}$ preferably are of 2 to 14, especially of 8 to 12, particularly of 10 carbon atoms. They are preferably straight-chained.

An even more preferred group of compounds of formula I is the compounds of formula Is

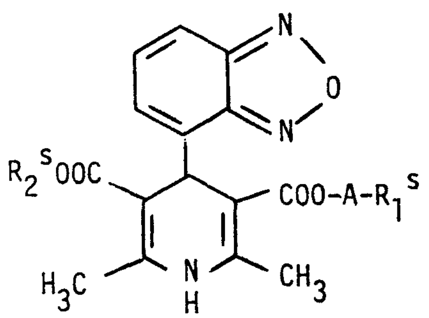

Is

wherein

$R_2^s$ is alkyl of 1 to 6 carbon atoms,

A is alkylene of 2 to 16 carbon atoms separating the carbonyl part from $R_1^s$ by at least 2 carbon atoms and

$R_1^s$ is a) a group $-(O)_m-$ 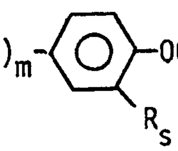 $-OCH_2CH(OH)CH_2NH-R_s^s$

wherein

m is 0 or 1,

$R_s$ is hydrogen or cyano and

$R_s^s$ is alkyl of 1 to 7 carbon atoms or

b) a group $-NHCH_2CH(OH)CH_2O-$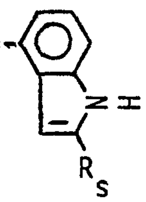,

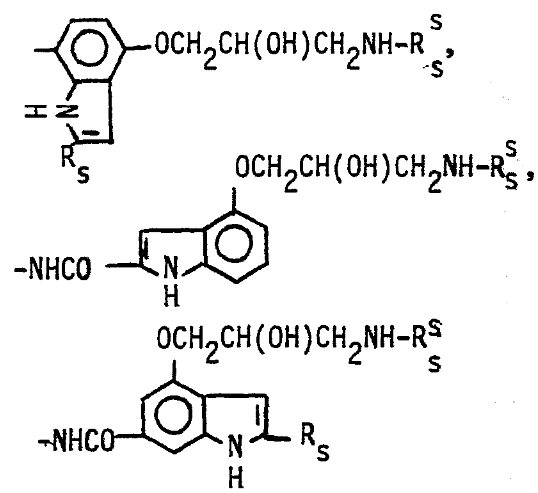, or

wherein $R_s$ and $R_s^s$ are as defined above.

$R_2^s$ preferably is of 1 to 4 carbon atoms, it especially is methyl or isopropyl.

m preferably is 1. $R_s$ preferably is cyano. $R_s^s$ preferably is of 3 to 7 carbon atoms, it preferably is branched in the α-position, it especially is isopropyl or tert-butyl, especially tert-butyl.

$R_1^s$ preferably is a group b).

A preferably is of 2 to 14, especially of 8 to 12, particularly of 10 carbon atoms. It preferably is straight-chained.

A compound of the invention may be obtained by a process comprising condensing a corresponding 4-aryl-1,4-dihydropyridine-3-and/or 5-carboxylic acid ester derivative having an appropriate reactive group in place of the β-blocker moiety, or a precursor thereof, with a corresponding, reactive β-blocker, or a precursor thereof.

In particular a compound of formula I may be obtained by a process comprising condensing a corresponding compound of formula II

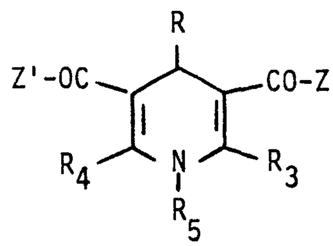

II

wherein R, $R_3$, $R_4$ and $R_5$ are as defined above,

Z is a reactive group and

Z' is a reactive group or has the significance indicated above for $-OR_2$,

or a precursor thereof,

with a corresponding, reactive β-blocker, or a precursor thereof.

The process of the invention may be effected in a manner analogous to known processes.

Condensation preferably is effected at elevated temperature, e.g. of about 80° to about 120°C. The reaction appropriately is effected in a solvent such as dimethylformamide or dioxane or in a melt.

The choice of the most appropriate condensation process variant depends of course on the nature of e.g. substituents $R_1$ and $R_2$.

A process variant may e.g. be an esterification. A reactive group, e.g. a group Z or Z' in formula II, is then conveniently 1H-imidazol-1-yl.

When the reactive β-blocker has a phenolic hydroxy substituent esterification thereof may be effected, e.g. with a compound of formula II wherein a reactive group Z or Z' is conveniently e.g. a group $-O-A_a-Z''$ wherein $A_a$ is alkylene and Z'' is chlorine, bromine or a group $R_z-SO_2-O-$ wherein $R_z$ is phenyl, tolyl or lower alkyl, preferably methyl. Z'' preferably is mesyloxy. The reactive β-blocker preferably is in the form of an alkaline metal salt.

A further condensation variant suitable for preparing compounds wherein the β-blocker moiety is a moiety 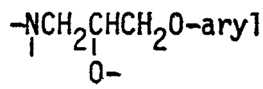 is e.g. by reacting a compound of formula II wherein a group Z or Z' is e.g. a group $-O-A_a-Z'''$ wherein $A_a$ is as defined above and Z''' is primary amino, with a compound of formula $R_zCH_2O$-aryl wherein $R_z$ is a group capable of reacting with a primary amine to give a 2-amino-1-hydroxyethyl group. $R_z$ may be a group of formula 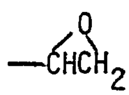 or a derivative of this group, e.g. a group of formula $-CH(OH)CH_2L$ wherein L is chlorine, bromine or a group $R_ySO_2O-$ wherein $R_y$ is phenyl, tolyl or lower alkyl. L is especially chlorine. $R_z$ preferably is a group of formula 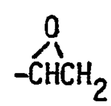.

Precursors of the starting materials are e.g. compounds in which a potentially reactive group is present in protected form. When potentially reactive groups are present, e.g. hydroxy or primary or secundary amino, it may be indicated to effect the condensation with such groups in protected form, e.g. for phenolic hydroxy in the form of a benzyloxy group, or for aliphatic hydroxy in the form of a tetrahydropyranyloxy group, or for amino in the form of an acylamino or a phthalimido group and to convert thereafter any

protecting group present as such into the desired substituent, e.g. benzyloxy to hydroxy, e.g. hydrogenolytically; tetrahydropyranyloxy to hydroxy, e.g. by acid hydrolysis; and protected amino to deprotected amino, e.g. by acid hydrolysis or hydrazinolysis. The $-\underset{\text{O}-}{\underset{|}{\text{C}}}\text{HCH}_2\underset{|}{\text{N}}-$ part of a β-blocker moiety may e.g. be protected in the form of an oxazolidine group, e.g. as a corresponding phenyloxazolidine. Deprotection is then preferably effected by acid hydrolysis.

A compound of the invention may be isolated from the reaction mixture and purified in a manner analogous to known methods.

A compound of the invention may exist in free form or where appropriate in salt form. A free form may be converted into a salt form in conventional manner and vice-versa. Suitable acids for acid addition salt formation include hydrochloric, malonic, p-toluene sulfonic and methanesulfonic acid.

When the substituents in the 2 and 6 positions and/or in the 3 and 5 positions of the 1,4-dihydropyridinyl moiety are different the carbon atom in the 4 position is asymmetrically substituted. The carbon atom in a β-blocker moiety which carries the oxy group is also asymmetrically substituted. A corresponding compound of the invention may thus exist in the racemic or in diastereoisomeric individual enantiomer form. Individual optical isomer forms may be obtained in conventional manner, e.g. by using corresponding optically active starting materials or by optical fractionation, e.g. by fractional crystallization.

A compound used as a starting material may be obtained in conventional manner.

Insofar as the preparation of any - - - - - - starting material is not particularly described, this is known or its preparation may be effected in conventional manner or in analogous manner to that described herein.

In the following Examples all temperatures are in degrees Centigrade and are uncorrected.

Example 1: (+)-(4R)-4-(2,1,3-benzoxadiazol-4-yl)-1,4-dihydro-5-isopropoxycarbonyl-2,6-dimethyl-3-pyridincarboxylic acid-10-[(2S)-3-(2-cyano-1H-indol-4-yloxy)-2-hydroxy-propylamino]decyl ester

1.9 g (+)-(4R)-4-(2,1,3-benzoxadiazol-4-yl)-1,4-dihydro-5-isopropoxycarbonyl-2,6-dimethyl-3-pyridincarboxylic acid-(10 -amino-decyl) ester and 0.4 g (+)-(S)-4-(2,3-epoxypropoxy)-1H-indol-2-carbonitrile are dissolved in 30 ml of methylene chloride, the solution is evaporated under reduced pressure and the residue is heated for 10 minutes at 70°. The product is chromatographed over silicagel using methylene chloride-methanol (saturated with $NH_3$) (19:1) as an eluent. The title compound is obtained [amorphous (foam); $[\alpha]_D^{20}$ = +14°; $[\alpha]_{546}^{20}$ = +20°, c = 0.8 g/dl in ethanol].

The epoxide used as a starting material is obtained as follows:

a) 80 g (S)-2,2-dimethyl-1,3-dioxolan-4-methanol dissolved in dimethylformamide are reacted at 0° with potassium hydroxide and thereafter with benzyl bromide. (S)-4-Benzyloxymethyl-2,2-dimethyl-1,3-dioxolan is obtained (clear oil; $[\alpha]_D^{20}$ = +9.6°, c = 2 % in methanol).

b) 93,3 g of the above product in hydrochloric acid aquous solution and acetone are reacted under refluxing for 2 hours. (R)-3-Benzyloxypropan-1,2-diol is obtained (colourless oil; $[\alpha]_D^{20}$ = -1.2°, c = 2 % in methanol).

c) 118 g of the above product in pyridine are reacted at 0° dropwise with 126.5 g of p-toluene sulfonic acid chloride in benzene and the mixture is stirred for 72 hours at room temperature. (S)-1-Benzyloxy-3-tosyloxy-2-propanol is obtained (oil, $[\alpha]_D^{20}$ = +8.3°, c = 2% in methanol).

d) 41.5 g of 4-Hydroxy-1H-indol-2-carboxamide are converted with sodium hydride into the corresponding sodium salt and this salt is reacted in dimethyl formamide with 87.8 g of the product obtained under c). The mixture is stirred for 40 hours at 100° oil bath temperature. After working up and chromatographic purification over silicagel (S)-4-(3-Benzyloxy-2-hydroxypropoxy)-1H-indol-2-carboxamide is obtained (M.P. 115-117°; $[\alpha]_D^{20}$ = -1.5°, c = 2 % in methanol).

e) 62.2 g of the above product are hydrogenated for 6 hours with palladium 10% on charcoal in methanol. (S)-4-(2,3-Dihydroxypropoxy)-1H-indol-2-carboxamide is obtained (M.P. 183-185°; $[\alpha]_D^{20}$ = +6.15°, c = 2 % in methanol).

f) 36.65 g of the above product are dissolved in pyridine and reacted for 1 hour at -15° to -5° with a solution of p-toluenesulfonic acid chloride in pyridine and the mixture stirred for 3 hours at 0°. (R)-4-(2-hydroxy-3-tosyloxypropoxy)-1H-indol-2-carboxamide is obtained (M.P. 162-168°; $[\alpha]_D^{20}$ = -13.5°, c = 2 % in methanol).

g) A solution of 44.2 g of the above product in methanol/tetrahydrofurane (1:1) is added dropwise at 0° to a solution of 2.76 g sodium in methanol over 1 1/2 hours and stirred for one hour. (S)-4-(2,3-Epoxypropoxy)-1H-indol-2-carboxamide is obtained (M.P. 125-135°; $[\alpha]_D^{20}$ = +26°, c = 2 % in methanol).

h) 7.9 g of the above product are suspended in dioxane and pyridine and a solution of 7.8 ml trifluoroacetic acid anhydride in dioxan is added thereto at 10° over 1 hour and the mixture is stirred for another hour. (S)-4-(2,3-Epoxypropoxy)-1H-indol-2-carbonitrile is obtained (M.P. 123-125°; $[\alpha]_D^{20}$ = +40.0°, c = 1 % in methanol).

Example 2: (-)-(4S)-4-(2,1,3-benzoxadiazol-4-yl)-1,4-dihydro-5-isopropoxycarbonyl-2,6-dimethyl-3-pyridincarboxylic acid-10-[(2S)-3-(2-cyano-1H-indol-4-yloxy)-2-hydroxy-propylamino]decyl ester

The title compound is obtained in a manner analogous to Example 1, starting from the corresponding (-)-(4S)-10-aminodecyl ester and is characterized as follows: amorphous; $[\alpha]_D^{20}$ = -20°; $[\alpha]_{546}^{20}$ = -27°, c = 0.9 g/dl in ethanol.

Example 3: (4S)-4-(2,1,3-benzoxadiazol-4-yl)-1,4-dihydro-5-methoxycarbonyl-2,6-dimethyl-3-pyridincarboxylic acid-8-(4-[(2RS,5S)-3-tert-butyl-2-phenyloxazolidin-5-yl-methoxy]-1H-indol-2-ylcarbonylamino)octyl ester

1.9 g (+)-(R)-4-(2,1,3-benzoxadiazol-4-yl)-1,4-dihydro-5-(1H-imidazol-1-ylcarbonyl)-2,6-dimethyl-3-pyridincarboxylic acid methyl ester and 2.6 g 4-[(2RS,5S)-3-tert-butyl-2-phenyloxazolidin-5-ylmethoxy]-1H-indol-2-carboxylic acid-(8-hydroxyoctyl)amide are heated 20 hours to 120° in 5 ml of dioxane. The dioxane is then distilled off and the mixture further heated at 120° for 5 hours. The product is chromatographed over silicagel using methylenechloride / ethanol (49:1) as an eluent. The title compound is obtained (amorphous; $R_f$ = 0.4, silicagel-plate, methylene chloride-ethanol 19:1).

The 8-hydroxyoctyl amide-oxazolidine (M.P. of the hydrogen malonate salt form 139-141° [dec.] ) used as a starting material is obtained according to the following reaction sequences:

1. 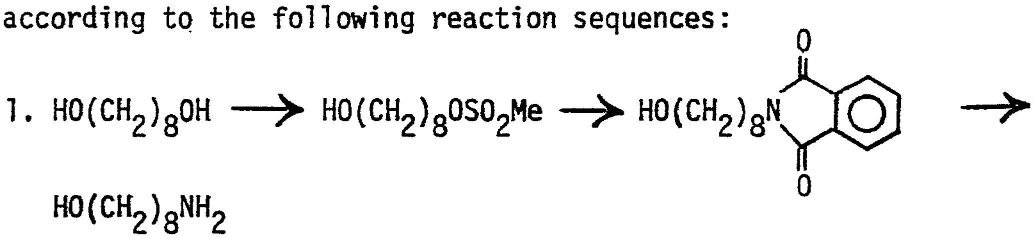

2. 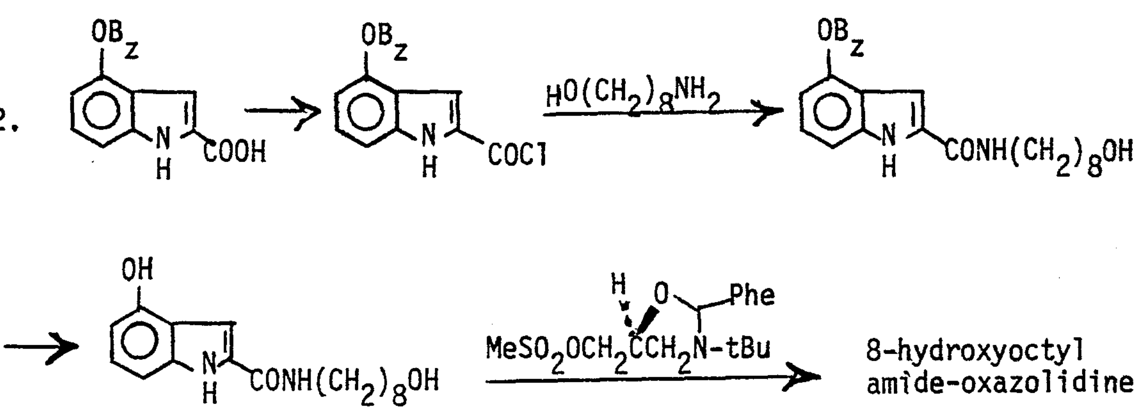

Example 4: (4R)-4-(2,1,3-benzoxadiazol-4-yl)-1,4-dihydro-5-methoxycarbonyl-2,6-dimethyl-3-pyridincarboxylic acid-8-(4-[(2RS,5S)-3-tert-butyl-2-phenyloxazolidin-5-yl-methoxy]-1H-indol-2-ylcarbonylamino)octyl ester

The title compound is obtained in a manner analogous to Example 3, starting from the corresponding (-)-(S)-imidazolide.

Example 5: (+)-(4S)-4-(2,1,3-benzoxadiazol-4-yl)-1,4-dihydro-5-methoxycarbonyl-2,6-dimethyl-3-pyridine carboxylic acid-8-{4-[(2S)-3-tert-butylamino-2-hydroxypropoxy]-1H-indol-2-ylcarbonylamino} octyl ester

(deprotection)

2.3 g of the title compound of Example 3 are heated for 20 minutes at 60° in 12 ml acetic acid and 12 ml water. The solution is then made alkaline by dropwise addition of concentrated sodium hydroxide solution under refrigeration and extracted with methylene chloride. The organic phase is dried over magnesium sulfate, the solvent evaporated to dryness under reduced pressure and the residue chromatographed over silicagel using methylene chloride-ethanol (saturated with ammonia) (93:7) as an eluent. The title compound is obtained (amorphous; $[\alpha]_D^{20} = +19°$, $[\alpha]_{546}^{20} = +28°$, c = 1.0 g/dl in ethanol).

Example 6: (-)-(4R)-4-(2,1,3-benzoxadiazol-4-yl)-1,4-dihydro-5-methoxycarbonyl-2,6-dimethyl-3-pyridine carboxylic-acid-8-{4-[(2S)-3-tert-butylamino-2-hydroxypropoxy]-1H-indol-2-ylcarbonylamino}octyl ester

(deprotection)

The title compound is obtained in a manner analogous to Example 5, starting from the title compound of Example 4, and is characterized as follows: amorphous; $[\alpha]_D^{20}$ = -22°, $[\alpha]_{546}^{20}$ = -31°, c = 1.1 g/dl in ethanol).

Example 7: (+)-(4R)-4-(2,1,3-benzoxadiazol-4-yl)-1,4-dihydro-5-isopropoxycarbonyl-2,6-dimethyl-3-pyridincarboxylic acid-2-[3-cyano-4-{(2S)-3-tert-butylamino-2-hydroxypropoxy}-phenoxy]ethyl ester

To a suspension of 0.52 g sodium hydride (50% w/v in oil) in 30 ml dimethylformamide is added 1.5 g (-)-(S)-2-(3-tert-butylamino-2-hydroxypropoxy)-5-hydroxybenzonitrile in hydrochloride form and the mixture is stirred for 1 hour at 50°. Then a solution of 2.4 g (+)-(4R)-4-(2,1,3-benzoxadiazol-4-yl)-1,4-dihydro-5-isopropoxycarbonyl-2,6-dimethyl-3-pyridincarboxylic acid-(2-methansulfonyloxyethyl) ester in 20 ml dimethylformamide is added and the solution stirred for 20 hours at 80°. After evaporation of the solvent under reduced pressure ice-water is added and the solution is extracted with ether. Then the ether solution is dried, concentrated under reduced pressure, and the residue is chromatographed using methylene chloride-methanol saturated with ammonia (19:1) as an eluent. The product is amorphous (foam); $[\alpha]_D^{20}$ = +25°, $[\alpha]_{546}^{20}$ = +31° (chloroform, c = 1 g/dl).

Example 8: (-)-(4S)-4-(2,1,3-benzoxadiazol-4-yl)-1,4-dihydro-5-isopropoxycarbonyl-2,6-dimethyl-3-pyridine carboxylic-acid-2-[3-cyano-4-{(2S)-3-tert-butylamino-2-hydroxy-propoxy}-phenoxy]ethyl ester

The title compound is obtained in a manner analogous to Example 7, starting from the corresponding (-)-(4S)-mesylate and is characterized as follows: (amorphous); $[\alpha]_D^{20} = -35°$; $[\alpha]_{546}^{20} = -42°$ (c = 1 g/dl, chloroform).

The following compounds of formula I are obtained in analogous manner:

| Ex. No. | Analogous to Ex.No. | R | Configuration at 4 position of 1,4-dihydro-pyridine part | | $R_1$ | Configuration at 2-position of amino-propoxy part | $R_2$ | $R_3$ | $R_4$ | $R_5$ |
|---|---|---|---|---|---|---|---|---|---|---|
| 9 | 3[a)] | 2,1,3-benzoxa-diazol-4-yl | (S) | | 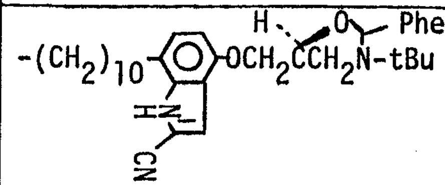 | (S) | Me | Me | Me | H |
| 10 | 4[a)] | id. | (R) | | id. | (S) | Me | Me | Me | H |
| 11 | 5;3[a')] | id. | (S) | (+) | 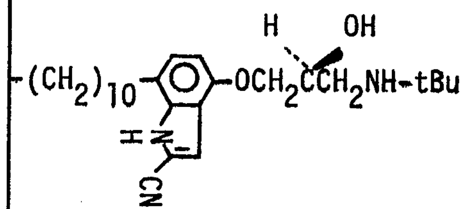 | (S) | Me | Me | Me | H |
| 12 | 6;4[a')] | id. | (R) | (-) | id. | (S) | Me | Me | Me | H |
| 13 | 5;7;3[b')] | id. | (R) | (+) | 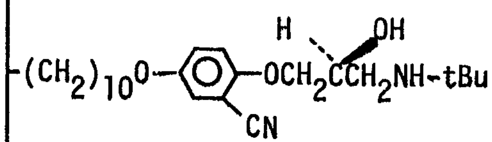 | (S) | iPr | Me | Me | H |
| 14 | 6;8;4[b')] | id. | (S) | (-) | id. | (S) | iPr | Me | Me | H |
| 15 | 3[b)];7 | id. | (R) | | 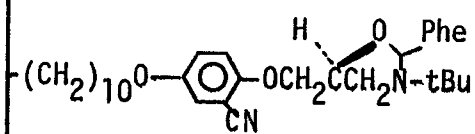 | (S) | iPr | Me | Me | H |
| 16 | 4[b)];8 | id. | (S) | | id. | (S) | iPr | Me | Me | H |

| Ex. No. | Analogous to Ex.No. | R | Configuration at 4 position of 1,4-dihydro-pyridine part | | $R_1$ | Configuration at 2 position of amino-propoxy part | $R_2$ | $R_3$ | $R_4$ | $R_5$ |
|---|---|---|---|---|---|---|---|---|---|---|
| 17 | 5;3[c)] | id. | (S) | (+) | 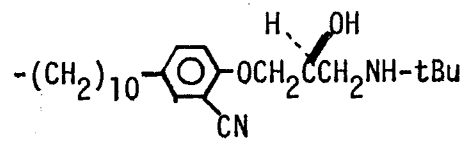 | (S) | Me | Me | Me | H |
| 18 | 6;4[c)] | id. | (R) | (-) | id. | (S) | Me | Me | Me | H |
| 19 | 3[d)] | id. | (S) | | 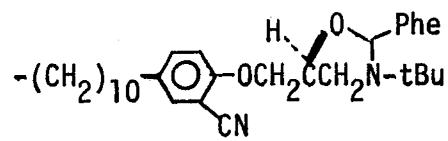 | (S) | Me | Me | Me | H |
| 20 | 4[d)] | id. | (R) | | id. | (S) | Me | Me | Me | H |
| 21 | 5;3e) | id. | (S) | (+) | 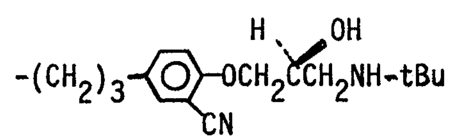 | (S) | Me | Me | Me | H |
| 22 | 6;4[e)] | id. | (R) | (-) | id. | (S) | Me | Me | Me | H |
| 23 | 3[f)] | id. | (S) | | 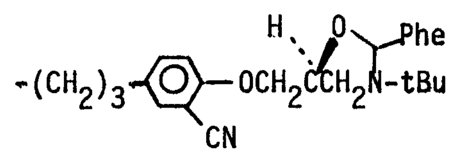 | (S) | Me | Me | Me | H |
| 24 | 4[f)] | id. | (R) | | id. | (S) | Me | Me | Me | H |

| Ex. No. | Analogous to Ex.No. | R | Configuration at 4 position of 1,4-dihydro-pyridine part | | $R_1$ | Configuration at 2 position of amino propoxy part | $R_2$ | $R_3$ | $R_4$ | $R_5$ |
|---|---|---|---|---|---|---|---|---|---|---|
| 25 | 5;3[g)] | 2,1,3-benzoxadiazol-4-yl | (S) | (+) | 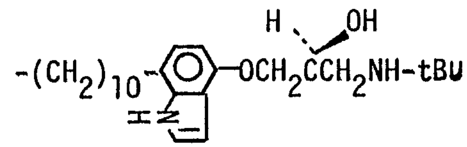 | (S) | Me | Me | Me | H |
| 26 | 6;4[g)] | id. | (R) | (-) | id. | (S) | Me | Me | Me | H |
| 27 | 3[h)] | id. | (S) | | 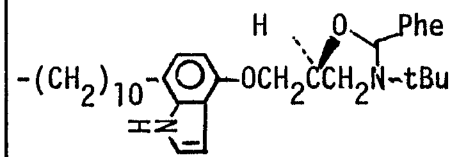 | (S) | Me | Me | Me | H |
| 28 | 4[h)] | id. | (R) | | id. | (S) | Me | Me | Me | H |
| 29 | 5;3[i)] | id. | (S) | (+) | 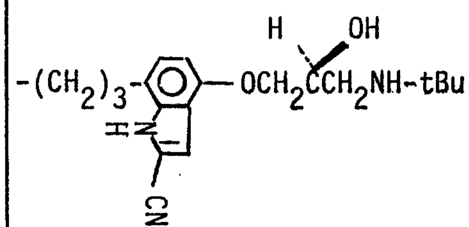 | (S) | Me | Me | Me | H |
| 30 | 6;4[i)] | id. | (R) | (-) | id. | (S) | Me | Me | Me | H |
| 31 | 3[j)] | id. | (S) | | 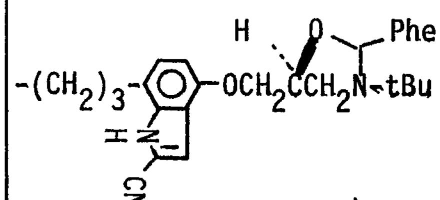 | (S) | Me | Me | Me | H |
| 32 | 4[j)] | id. | (R) | | id. | (S) | Me | Me | Me | H |

| Ex. No. | Analogous to Ex.No. | R | Configuration at 4 position of 1,4-dihydro-pyridine part | R1 | Configuration at 2 position of amino propoxy part | R2 | R3 | R4 | R5 |
|---|---|---|---|---|---|---|---|---|---|
| 33 | 5;3[k]; 7[m] | 2,1,3-benzoxa-diazol-4-yl | (S) (+) | 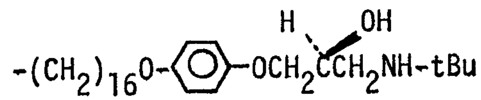 | (S) | Me | Me | Me | H |
| 34 | 6;4[k]; 8[m] | id. | (R) (-) | id. | (S) | Me | Me | Me | H |
| 35 | 3[l];7[n] | id. | (S) | 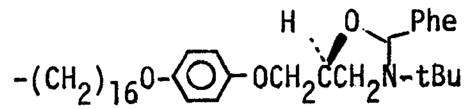 | (S) | Me | Me | Me | H |
| 36 | 4[l];8[n] | id. | (R) | id. | (S) | Me | Me | Me | H |
| 37 | 5;3[o] | id. | (S) (+) | 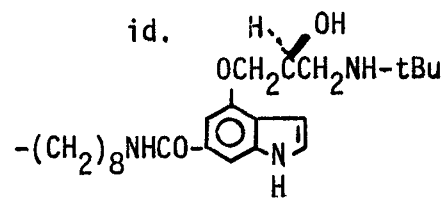 | (S) | Me | Me | Me | H |
| 38 | 6;4[o] | id. | (R) (-) | id. | (S) | Me | Me | Me | H |
| 39 | 3[p] | id. | (S) | 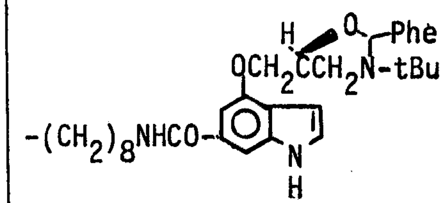 | (S) | Me | Me | Me | H |
| 40 | 4[p] | id. | (R) | id. | (S) | Me | Me | Me | H |

| Ex. No. | Analogous to Ex.No. | R | Configuration at 4 position of 1,4-dihydro-pyridine part | | R1 | Configuration at 2 position of amino propoxy part | R2 | R3 | R4 | R5 |
|---|---|---|---|---|---|---|---|---|---|---|
| 41 | 3 | 2,1,3-benzoxa-diazol-4-yl | (R) | (+) | 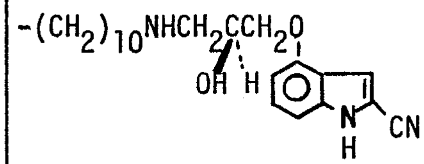 | (S) | iPr | Me | Me | H |
| 42 | 4 | id. | (S) | (-) | id. | (S) | iPr | Me | Me | H |
| 43 | 3[r] | id. | (S) | (+) | 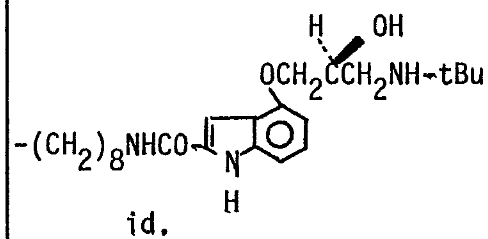 | (S) | Me | Me | Me | H |
| 44 | 4[r] | id. | (R) | (-) | id. | (S) | Me | Me | Me | H |
| 45 | 5;3[q] | id. | (R) | (+) | 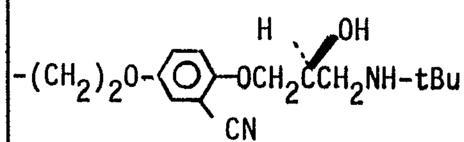 | (S) | iPr | Me | Me | H |
| 46 | 6;4[q] | id. | (S) | (-) | id. | (S) | iPr | Me | Me | H |
| 47 | 3[m];7[n] | id. | (R) | | 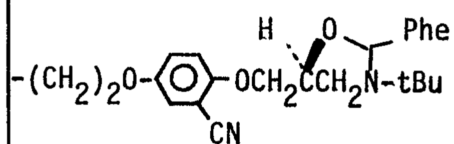 | (S) | iPr | Me | Me | H |
| 48 | 4[m];8[n] | id. | (S) | | id. | (S) | iPr | Me | Me | H |

| Ex. No. | Analogous to Ex.No. | R | Configuration at 4 position of 1,4-dihydropyridine part | $R_1$ | Configuration at 2 position of amino propoxy part | $R_2$ | $R_3$ | $R_4$ | $R_5$ |
|---|---|---|---|---|---|---|---|---|---|
| 49 | 1[s]);3 | 2,1,3-benzoxadiazol-4-yl | (S) (+) | $-(CH_2)_{10}$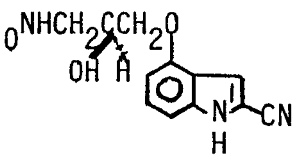 | (S) | Me | Me | Me | H |
| 50 | 2[s]);4 | id. | (R) (-) | id. | (S) | Me | Me | Me | H |

Bz = benzyl
tBu = tert-butyl
Et = ethyl
iPr = isopropyl
Me = methyl
Phe = phenyl

b = in free base form
ch = in hydrochloride acid addition salt form.

Ex. = Example

a) Starting from 4-[(2RS,5S)-3-tert-butyl-2-phenyloxazolidin-5-ylmethoxy]-2-cyano-7-(10-hydroxy-decyl)-1H-indol (oil) — — — — — — obtained according to the following reaction sequence:

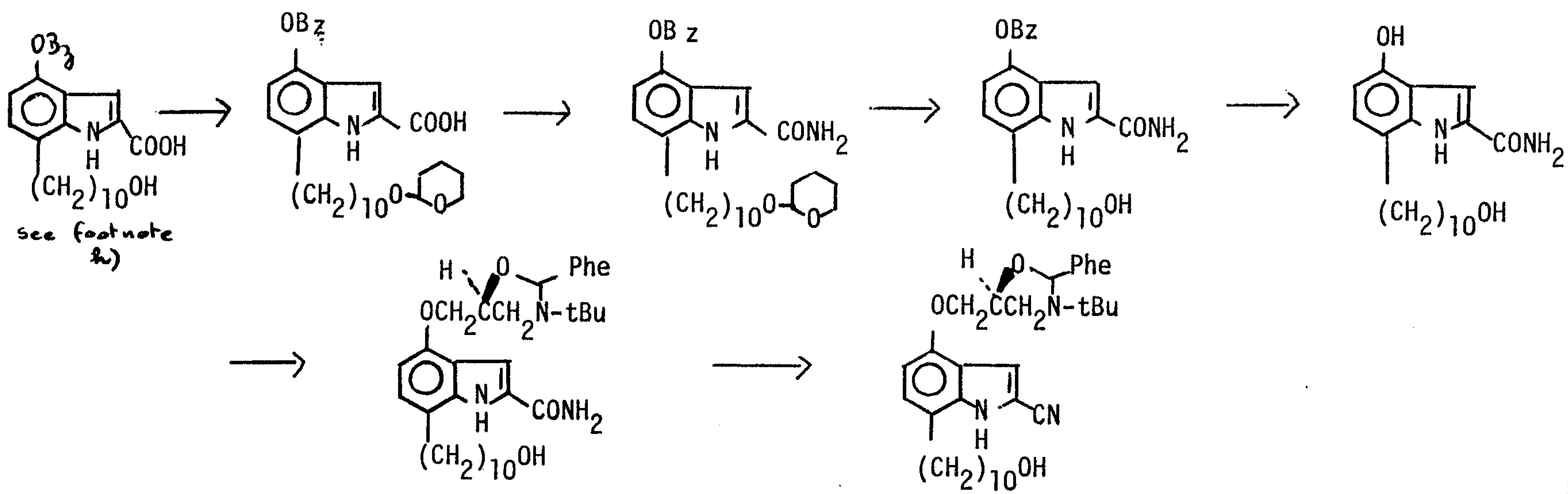

see footnote h)

a ') 4-[(2S)-3-tert-Butylamino-2-hydroxypropoxy]-2-cyano-7-(10-hydroxydecyl)-1H-indol (M.P. of the oxalate form 219°) is obtained by acid hydrolysis of the oxazolidine compound described under footnote a).

b) Starting from 2-[(2RS,5S)-3-tert-butyl-2-phenyloxazolidin-5-ylmethoxy]-5-(10-hydroxydecyl)benzonitrile obtained as follows:

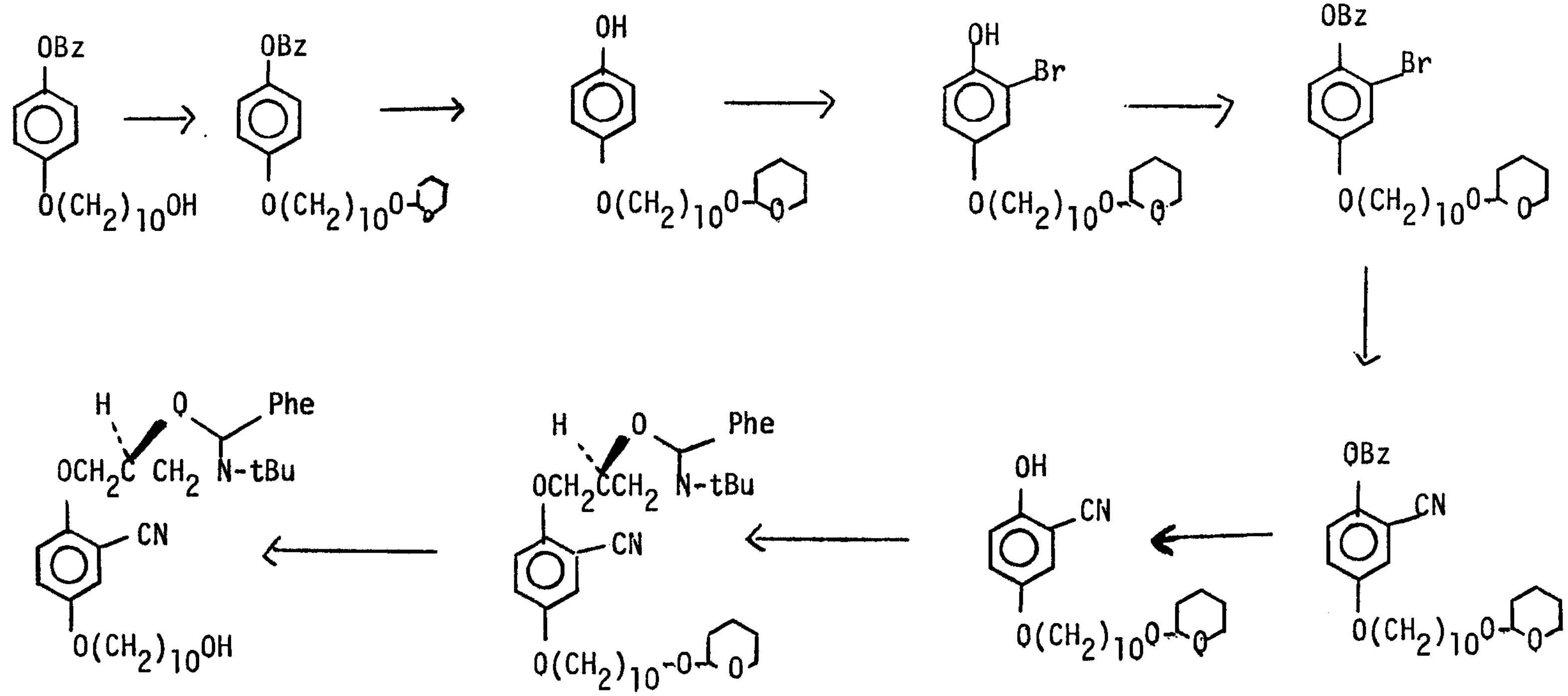

b') 2-[(2S)-3-tert-Butylamino-2-hydroxypropoxy]-5-hydroxy-benzonitrile is obtained by acid hydrolysis of the oxazolidine compound described under footnote b).

c) 2-[(2S)-3-tert-Butylamino-2-hydroxypropoxy]-5-(10-hydroxydecyl)-benzonitrile (M.P. of the hydrogen oxalate form 107-109°) is obtained by acid hydrolysis of the compound described under footnote d)

d) Starting from 2-[(2RS,5S)-3-tert-butyl-2-phenyloxazolidin-5-ylmethoxy]-5-(10-hydroxydecyl)-benzonitrile ( oil) - - - - - - - (=compound A) obtained by the following reaction sequence:

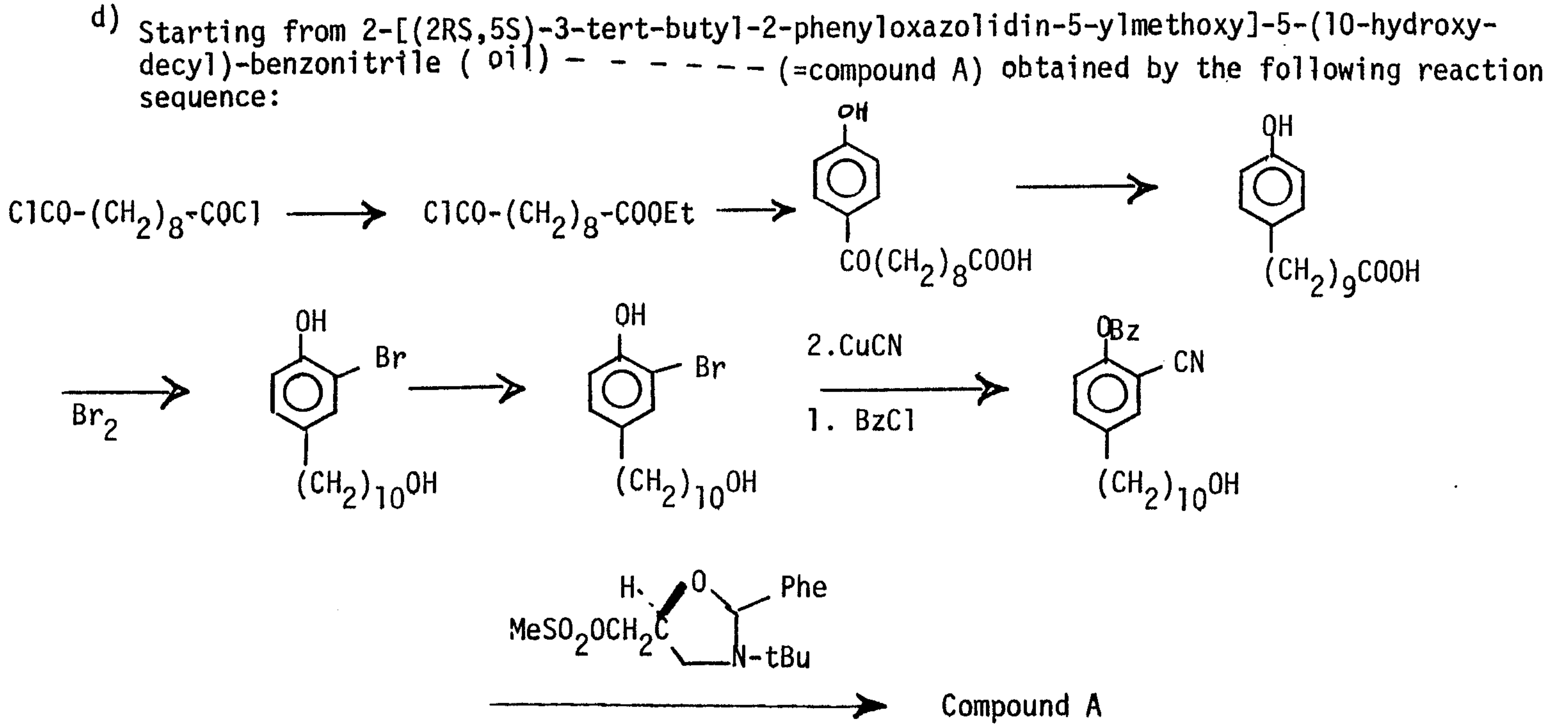


e) 2-[(2S)-3-tert-Butylamino-2-hydroxypropoxy]-5-(3-hydroxypropyl)-benzonitrile (M.P. of hydrogen oxalate form 108-111°) is obtained by acid hydrolysis of the compound described under footnote f).

f)Starting from 2-[(2RS,5S)-3-tert-Butyl-2-phenyloxazolidin-5-ylmethoxy]-5-(3-hydroxypropyl)-benzonitrile (oil) - - - - - - - - - - - - obtained by the following reaction sequence:

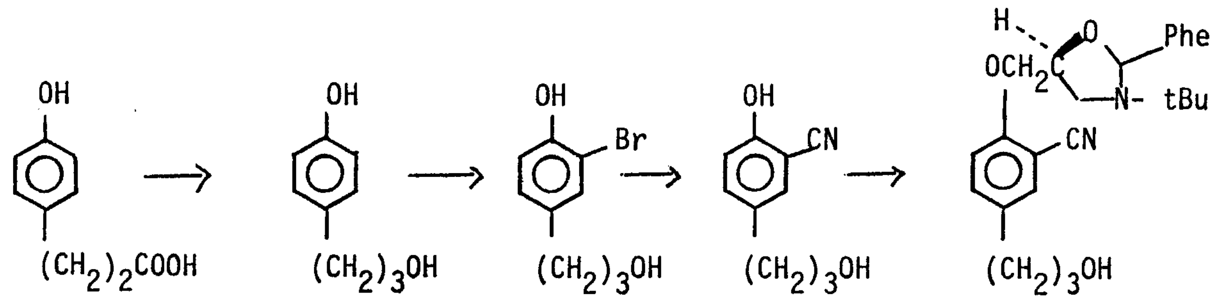

g)4-[(2S)-3-tert-Butylamino-2-hydroxypropoxy]-7-(10-hydroxydecyl)-1H-indol (M.P. of the hydrogen oxalate form 120-122°) is obtained by acid hydrolysis of the compound described under footnote h).

h)4-[(2RS,5S)-3-tert-Butyl-2-phenyloxazolidin-5-ylmethoxy]-7-(10-hydroxydecyl)-1H-indol (oil) is obtained by the following reaction sequence:

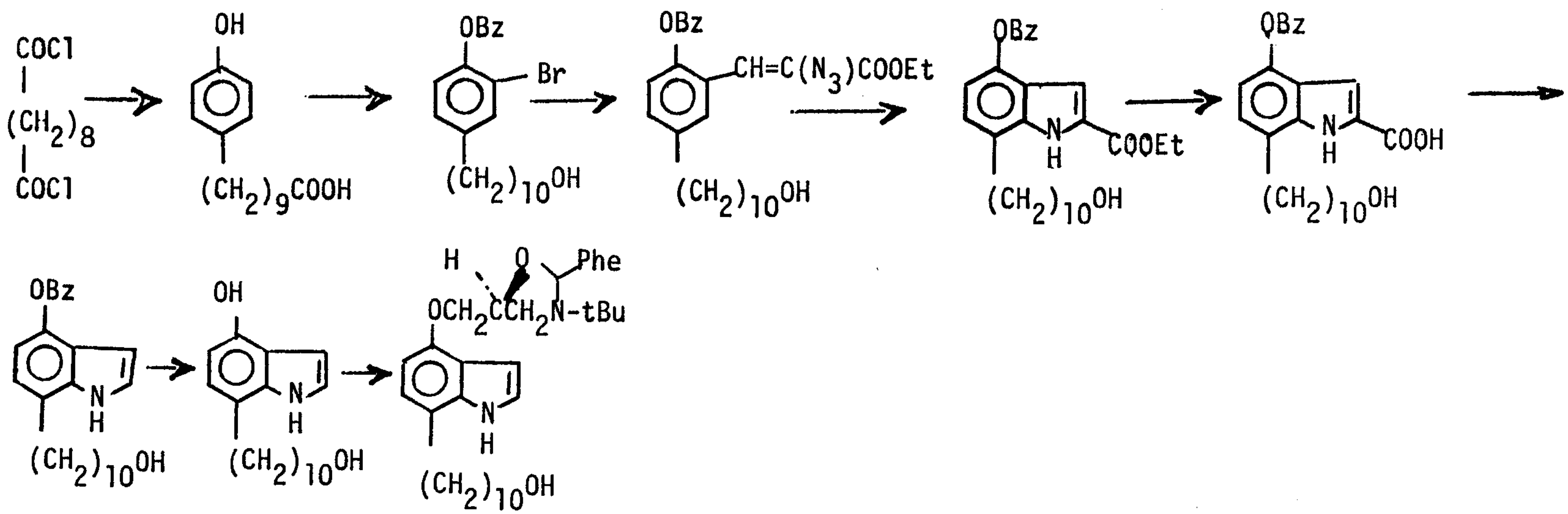

i) 4-[(2S)-3-tert-Butylamino-2-hydroxypropoxy]-2-cyano-7-(3-hydroxypropyl)-1H-indol (M.P. of the oxalate form 148-152°) is obtained by acid hydrolysis of the compound described under foonote j).

j) 4-[(2RS,5S)-3-tert-Butyl-2-phenyloxazolidin-5-ylmethoxy]-2-cyano-7-(3-hydroxypropyl)-1H-indol (oil) - - - - - - - is obtained according to the following reaction sequence:

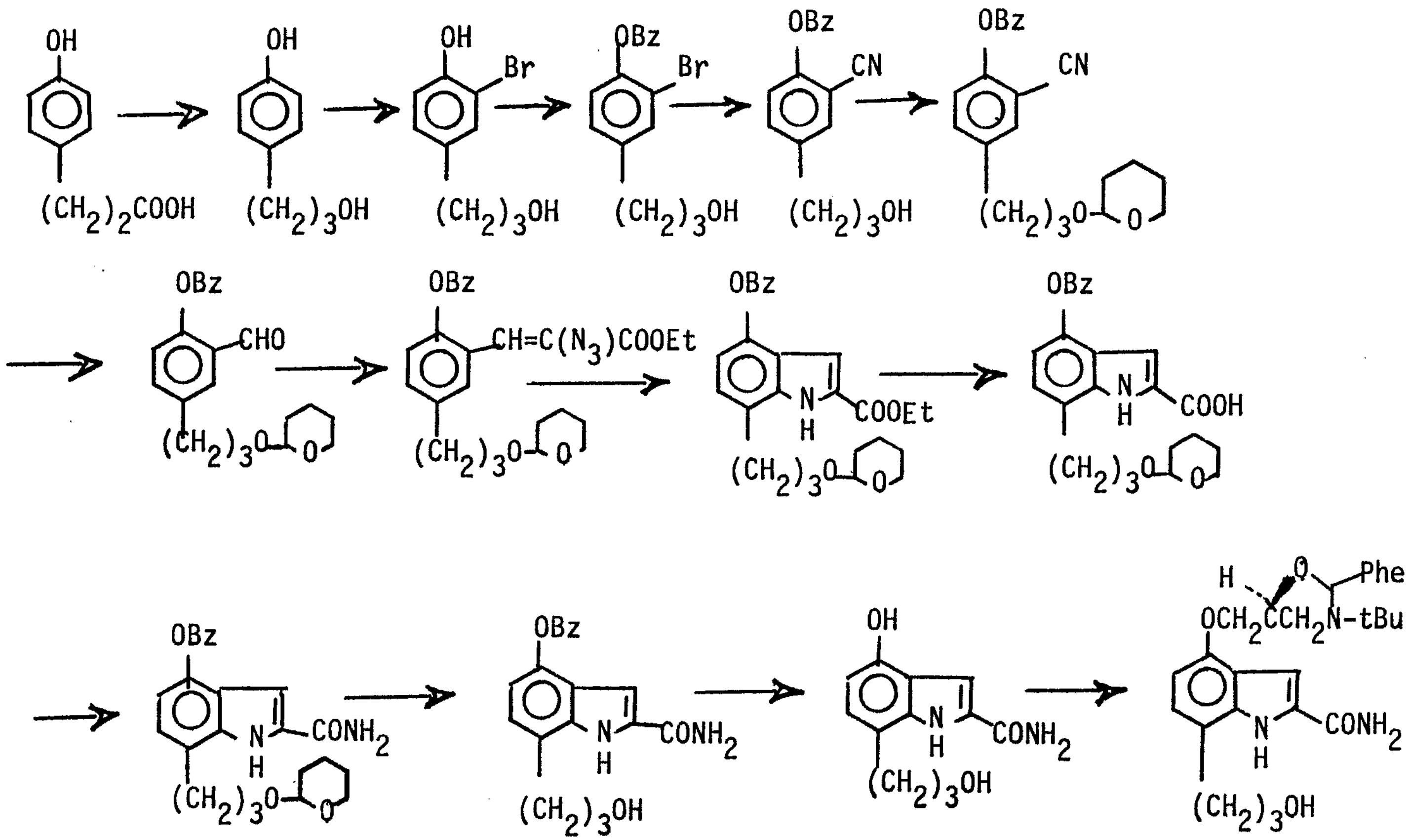

k) 16-{4-[(2S)-3-tert-Butylamino-2-hydroxypropoxy]-phenoxy}hexadecanol (M .P. 68-70°) is obtained by acid hydrolysis of the compound described under foonote l).

l) Starting from 4-[(2RS,5S)-3-tert-butyl-2-phenyloxazolidin-5-ylmethoxy]-1-(16-hydroxy-hexadecyl)benzol ( oil ) ———— ——— — obtained by the following reaction sequence:

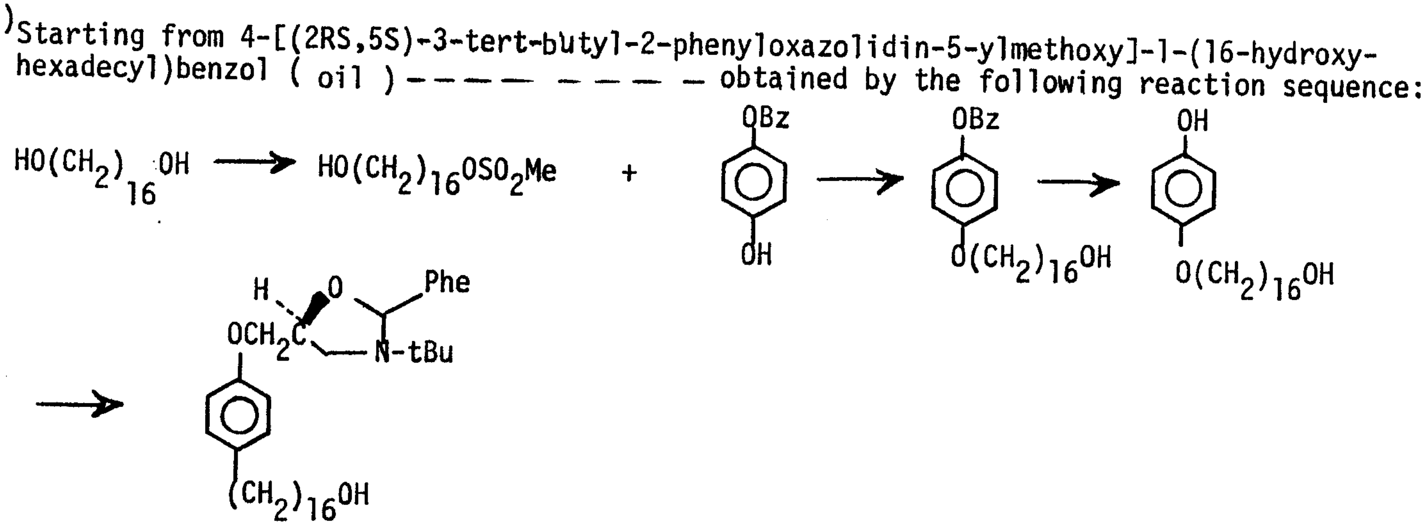

m) Starting from 2-[(2RS,5S)-3-tert-butyl-2-phenyloxazolidin-5-ylmethoxy]-5-(2-hydroxyethyl)-benzonitrile ( oil ) – —– · – ––– obtained by the reaction corresponding to that in footnote f).

n) 5-Hydroxy-2-[(2RS, 5S)-3-tert-butyl-2-phenyloxazolidin-5-ylmethyl]-benzonitrile ( oil ) used as a starting material is obtained by debenzylation of the benzyloxy-oxazolidine described in Example 7d).

o) 4-[(2S)-3-tert-Butylamino-2-hydroxypropoxy]-1H-indol-6-carboxylic acid-(8-hydroxyoctyl)-amide (M.P. of hydrogen malonate salt form 139-141° [dec.])is obtained by acid hydrolysis of the compound described under footnote p).

p) Starting from 4-[(2RS,5S)-3-tert-butyl-2-phenyloxazolidin-5-ylmethoxy]-1H-indol-6-carboxylic acid-(8-hydroxyoctyl)amide ( oil ) – ––– obtained as follows:

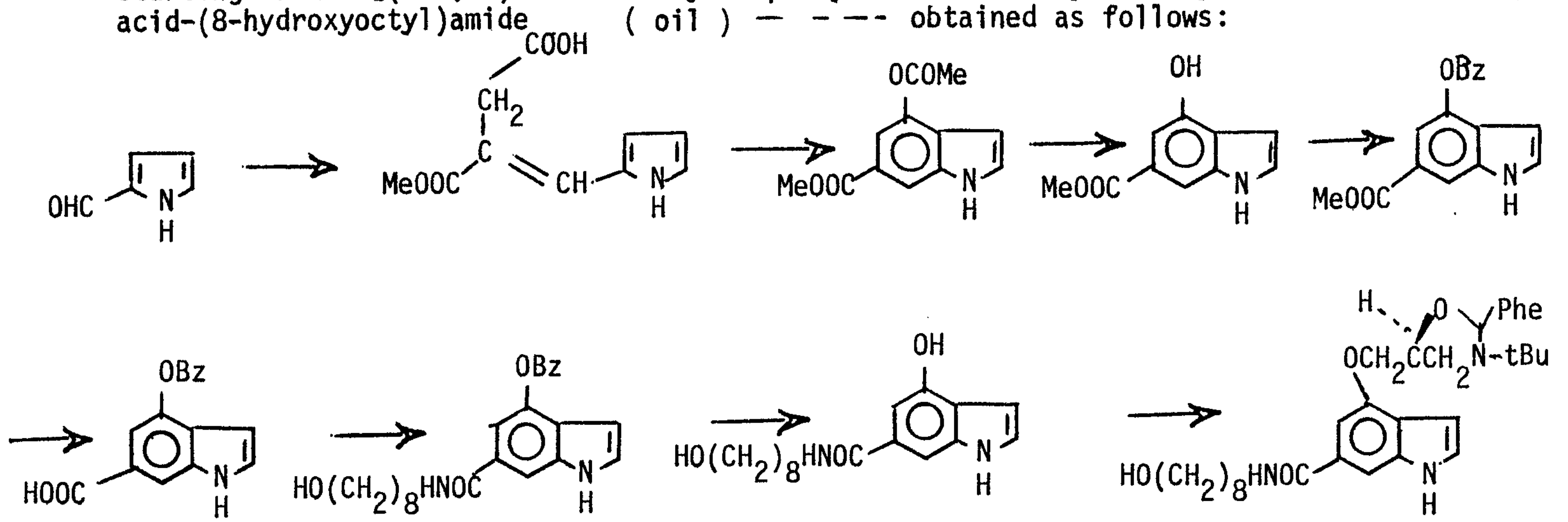

q) 2-[(2S)-3-tert-Butylamino-2-hydroxypropoxy]-5-(2-hydroxyethyl)-benzonitrile (oil) is obtained by acid hydrolysis of the compound described under footnote m).

r) 4-[(2S)-3-tert-Butylamino-2-hydroxypropoxy]-1H-indol-2-carboxylic acid-(8-hydroxyoctyl)amide (M.P. of oxalate salt form 192-194°) is obtained by acid hydrolysis of the 8-hydroxyoctyl amide-oxazolidine described in Example 3.

s) (+)-(4S)-4-(2,1,3-Benzoxadiazol-4-yl)-1,4-dihydro-5-methoxycarbonyl-2,6-dimethyl-3-pyridine carboxylic acid-(10-aminodecyl)ester (M.P. of free base form 130°; $[\alpha]_C^{20}$ = +33°; $[\alpha]_{546}^{20}$ = +48°, c = 0.8 g/dl in ethanol) and its corresponding (-)-(4R)-isomer are obtained by reacting (+)-(4S)-4-(2,1,3-benzoxadiazol-4-yl)-1,4-dihydro-5-methoxycarbonyl-2,6-dimethyl-3-pyridine carboxylic acid-(10-methanesulfonyloxydecyl)ester or, respectively, its (-)-(4R)-isomer with potassium phthalimide and reacting the resultant (10-phthalimidodecyl)ester with hydrazine hydrate.

Physical characterization data are as follows:

| Ex.No. | M.P. | Optical rotation $[\alpha]_D^{20}$ | $[\alpha]_{546}^{20}$ | Concentration (g/dl) | Solvent |
|---|---|---|---|---|---|
| 11 | b amorphous | +21° | +31° | 1.0 | ethanol |
| 12 | b amorphous | -20° | -28° | 1.1 | ethanol |
| 13 | b amorphous | +15° | +22° | 0.7 | $CHCl_3$ |
| 14 | b amorphous | -27° | -35° | 0.7 | $CHCl_3$ |
| 15 | b amorphous | | | | |
| 16 | b amorphous | | | | |
| 17 | b amorphous | +16° | +23° | 0.8 | ethanol |
| 18 | b amorphous | -24° | -34° | 0.6 | ethanol |
| 21 | b amorphous | +47° | +65° | 0.5 | ethanol |
| 22 | b amorphous | -49° | -65° | 0.5 | ethanol |
| 25 | b amorphous | +27° | +40° | 0.6 | ethanol |
| 26 | b amorphous | -26° | -36° | 0.4 | ethanol |

| Ex.No. | M.P. | Optical rotation $[\alpha]_D^{20}$ | $[\alpha]_{546}^{20}$ | Concentration (g/dl) | Solvent |
|---|---|---|---|---|---|
| 29 | b amorphous | +52° | +70° | 1.1 | ethanol |
| 30 | b amorphous | -52° | -70° | 1.0 | ethanol |
| 33 | ch amorphous | -13° | +20° | 1.0 | ethanol |
| 34 | ch amorphous | -28° | -38° | 1.1 | ethanol |
| 37 | b amorphous(foam) | +14.8° | +26.9° | 1.3 | ethanol |
| 38 | b amorphous(foam) | -23.4° | -32.5° | 1.7 | ethanol |
| 41 | see Example 1 | | | | |
| 42 | see Example 2 | | | | |
| 43 | see Example 5 | | | | |
| 44 | see Example 6 | | | | |
| 45 | see Example 7 | | | | |
| 46 | see Example 8 | | | | |
| 49 | b amorphous | + 19° | + 28° | 1.0 | ethanol |
| 50 | b amorphous | - 24° | - 35° | 1.1 | ethanol |

The compounds of the invention possess pharmacological activity. They are indicated for use as pharmaceuticals.

They possess dualistic activity as calcium antagonists and as β-adrenoceptor blocking agents.

The compounds thus exhibit effects typical of calcium antagonists. They exert a pronounced muscle-relaxing effect, particularly on smooth muscle, as evidenced by vasodilating and blood pressure lowering activity in standard tests. For example in the anaesthetized cat and rabbit tests using tracer microspheres (for methods see: R.P. Hof et al., Basic Res.Cardiol. 75 [1980] 747-756 and 76 [1981] 630-638; R.P. Hof et al., J.Cardiovasc.Pharmacol. 4 [1982] 352-362; R.P. Hof, Br.J.Pharmacol. 85 [1985] 75-87) coronary vasodilation, an increase in skelettal muscle blood flow and a fall in blood pressure are observed upon intravenous administration of from about 0.1 to about 1 mg/kg.

In the anaesthetized rat test a fall in blood pressure is also observed upon intravenous administration of from about 0.1 to about 1 mg/kg.

This test method is as follows:

Male Wistar rats are anaesthetized with 140 mg/kg thiobarbital, the trachea cannulated and the animals allowed to breathe normal room air. The femoral artery is cannulated for the recording of blood pressure and heart rate via a Statham pressure transducer and the drug is infused into the femoral vein over a 15-minute period in a total volume of 1 ml. Body temperature is maintained at 36°C. After an initial stabilization period of about 1 hour blood pressure and heart rate remain stable under these conditions for more than 6 hours, allowing the effects of drugs on these parameters to be monitored.

The compounds are longer acting than known standard calcium antagonistic compounds, they are suitable e.g. for once-a-day administration. The activity of the compounds of Examples 12 and 14 thus persists for at least 6 hours.

The compounds are therefore indicated for use as calcium antagonists for the prevention and treatment of

- coronary insufficiency, e.g. Angina pectoris;
- other vascular disturbances e.g. in limbs such as intermittent claudication and spasms, e.g. cholic;
- hypertension.

Further the compounds also possess β-adrenoceptor blocking activity as indicated by standard tests. In binding studies with 3-[$^{125}$I]-iodocyanopindolol with membranes prepared from left guinea pig ventricles (method of D. Hoyer et al., Naunyn-Schmiedeberg's Arch. Pharmacol. 318 [1982] 319-329), a displacement of the radioactive ligand is observed at a bath concentration of from about 3 x $10^{-9}$M to about $10^{-6}$M, indicative of affinity of the test compounds to β-receptors.

Corresponding activity is also observed in vivo e.g. in their ability to inhibit heart rate increases caused by injections of the β-adrenoceptor agonist isoprenaline in conscious rabbits at a dosage of from about 0.3 mg/kg to about 3.0 mg/kg i.v.

The compounds are therefore indicated for use as β-adrenoceptor blocking agents, e.g. for the prophylaxis and therapy of coronary diseases, such as angina pectoris, conditions which are associated with sympathetic over-stimulation, e.g. nervous heart complaints, myocardial infarction, hypertension and for the treatment of migraine, glaucoma and thyreotoxicosis. In view of their antiarrhythmic effect they are indicated for use as antiarrhythmics for the treatment of disturbances in the heart rhythm, such as supraventricular tachycardia.

It can be seen therefore that the compounds possess dualistic activity as calcium antagonists and β-adrenoceptor blocking agents, the calcium antagonistic activity originating from the 1,4-dihydropyridinyl component moiety and the β-adrenergic blocking activity from the β-blocker component moiety. Combination on one molecule has the advantage that bioavailability, metabolism and duration of activity for both active principles are harmonized, thus reducing interindividual variations,and that side effects commonly associated with calcium antagonists, such as tachycardia, are thereby suppressed by the β-blocking component.

It is surprising that combination of the two active principles retains the pharmacological activity of both separate components.

An indicated daily dosage is from about 0.3 mg to about 50 mg, suitably administered, e.g. orally, in divided dosages of from about 0.1 mg to about 25 mg of the compounds or in sustained release form.

The invention also provides a compound as defined above for use as a pharmaceutical. It also provides the use of a compound of the invention in the manufacture of a pharmaceutical composition for the treatment of a condition as mentioned above.

Of the compounds in optically active form those in which the carbon atom carrying the oxy group in the β-blocker moiety has the (S)-configuration are pharmacologically more active than the corresponding (R)-enantiomers.

The preferred uses of the compounds are the use against coronary diseases and hypertension, especially hypertension.

Preferred are the title compounds of Examples 1, 2, 5, 6 and 12, especially of Examples 1, 2 and 12.

The compounds of the invention may be administered in free form or where appropriate in pharmaceutically acceptable salt form, preferably acid addition salt form. Such salt forms exhibit the same order of activity as the free forms and are readily prepared in conventional manner. The present invention also provides a pharmaceutical composition comprising a compound of the invention in free form or where appropriate in pharmaceutically acceptable salt form, in association with a pharmaceutical carrier or diluent. Such compositions may be in the form of, for example, a capsule suitable e.g. for sublingual administration, or a tablet.

CLAIMS:

1. A 4-aryl-1,4-dihydropyridine-3- and/or 5-carboxylic acid ester derivative wherein at least one ester group is additionally substituted by a β-blocker moiety

with the proviso that it is other than a compound of formula Y

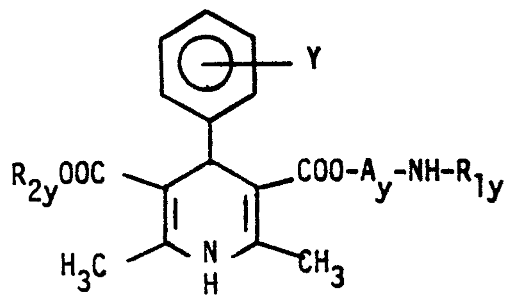

Y

wherein

either

Y is nitro,

$R_{1y}$ is 2-hydroxy-2-phenylethyl or 2-hydroxy-3-phenoxypropyl,

$R_{2y}$ is alkyl of 1 to 10 carbon atoms and

$A_y$ is alkylene of 1 to 10 carbon atoms optionally interrupted by an oxygen atom,

or

Y is hydrogen, nitro or trifluoromethyl,

$R_{1y}$ is 2-hydroxy-3-phenoxypropyl optionally substituted in the phenyl ring by halogen, lower alkyl, allyl, loweralkoxy (lower)-alkyl, allyloxy, cyano or ethinyl, or is 2-hydroxy-3-indolyl-oxypropyl,

$R_{2y}$ is lower alkyl and

$A_y$ is $-CH_2CR^yR^{y'}-$ wherein $R^y$ and $R^{y'}$ independently are hydrogen or methyl,

in free form or where appropriate in salt form.

2. A compound of formula I

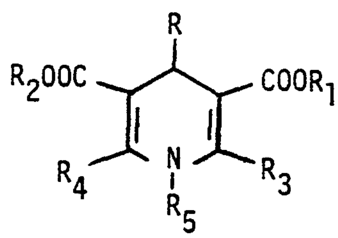

I

 0191724
100-6545

wherein

R is optionally substituted aryl,

$R_1$ is a group usual in the 4-aryl-1,4-dihydropyridine-3,5-dicarboxylic acid ester field but additionally substituted by a β-blocker moiety,

$R_2$ is a group usual in the 4-aryl-1,4-dihydropyridine-3,5-dicarboxylic acid ester field or independently has the significance indicated above for $R_1$ and

$R_3$, $R_4$ and $R_5$ independently are a group usual in the 4-aryl-1,4-dihydropyridine-3,5-dicarboxylic acid ester field,

with the proviso that it is other than a compound of formula Y as defined in claim 1,

in free form or where appropriate in salt form.

3. A compound of claim 1 selected from

(+)-(4R)-4-(2,1,3-benzoxadiazol-4-yl)-1,4-dihydro-5-isopropoxycarbonyl-2,6-dimethyl-3-pyridincarboxylic acid-10-[(2S)-3-(2-cyano-1H-indol-4-yloxy)-2-hydroxypropylamino]decyl ester,

(-)-(4S)-4-(2,1,3-benzoxadiazol-4-yl)-1,4-dihydro-5-isopropoxycarbonyl-2,6-dimethyl-3-pyridincarboxylic acid-10-[(2S)-3-(2-cyano-1H-indol-4-yloxy)-2-hydroxypropylamino]decyl ester and

(-)-(4R)-4-(2,1,3-benzoxadiazol-4-yl)-1,4-dihydro-5-methoxycarbonyl-2,6-dimethyl-3-pyridincarboxylic acid-10-(4-[(2S)-3-tert-butylamino-2-hydroxypropoxy]-2-cyano-1H-indol-7-yl)decyl ester (Example 12),

in free form or where appropriate in salt form.

4. A process for the preparation of a compound of claim 1 which comprises condensing a corresponding 4-aryl-1,4-dihydropyridine-3- and/or 5-carboxylic acid ester derivative having an appropriate reactive group in place of the β-blocker moiety, or a precursor thereof, with a corresponding, reactive β-blocker, or a precursor thereof.

5. A pharmaceutical composition comprising a compound of claim 1 in free form or where appropriate in pharmaceutically acceptable salt form in association with a pharmaceutical carrier or diluent.

6. A method of preventing or treating coronary insufficiency and coronary diseases such as Angina pectoris, other vascular disturbances e.g. in limbs, hypertension, conditions which are associated with sympathetic overstimulation, myocardial infarction, migraine, glaucoma, thyreotoxicosis or disturbances in the heart rythm which comprises administering to an animal in need of such treatment a therapeutically effective amount of a compound of claim 1 in free form or where appropriate in pharmaceutically acceptable salt form.

7. A compound of claim 1 for use as a pharmaceutical.

8. Use of a compound of claim 1 in the manufacture of a pharmaceutical composition for the treatment of a condition as defined in claim 5.

9. A compound selected from:

- (+)-(4S)-4-(2,1,3-benzoxadiazol-4-yl)-1,4-dihydro-5-methoxycarbonyl-2,6-dimethyl-3-pyridine carboxylic acid-(10-aminodecyl) ester and
- (-)-(4R)-4-(2,1,3-benzoxadiazol-4-yl)-1,4-dihydro-5-methoxycarbonyl-2,6-dimethyl-3-pyridine carboxylic acid-(10-aminodecyl) ester.

or -------------- selected from:

- 4-[(2S)-3-tert-butylamino-2-hydroxypropoxy]-2-cyano-7-(10-hydroxydecyl)-1H-indol
- 4-[(2S)-3-tert-butylamino-2-hydroxypropoxy]-5-hydroxybenzonitrile
- 2-[(2S)-3-tert-butylamino-2-hydroxypropoxy]-5-(10-hydroxydecyl) benzonitrile
- 2[(2S)-3-tert-butylamino-2-hydroxypropoxy]-5-(3-hydroxypropyl) benzonitrile
- 4-[(2S)-3-tert-butylamino-2-hydroxypropoxy]-7-(10-hydroxydecyl)-1H-indol
- 4-[(2S)-3-tert-butylamino-2-hydroxypropoxy]-2-cyano-7-(3-hydroxypropyl)-1H-indol
- 16-{4-[(2S)-3-tert-butylamino-2-hydroxypropoxy]phenoxy} hexadecanol
- 4-[(2S)-3-tert-butylamino-2-hydroxypropoxy]-1H-indol-6-carboxylic acid-(8-hydroxyoctyl)amide
- 2-[(2S)-3-tert-butylamino-2-hydroxypropoxy]-5-(2-hydroxyethyl) benzonitrile

- 4-[(2S)-3-tert-butylamino-2-hydroxypropoxy]-1H-indol-2-carboxylic acid-(8-hydroxyoctyl)amide,

or selected from:

- 4-[(2RS,5S)-3-tert-butyl-2-phenyloxazolidin-5-ylmethoxy]-1H-indol-2-carboxylic acid-(8-hydroxyoctyl)amide
- 4-[(2RS,5S)-3-tert-butyl-2-phenyloxazolidin-5-yl-methoxy]-2-cyano-7-(10-hydroxydecyl)-1H-indol
- 4-[(2RS,5S)-3-tert-butyl-2-phenyloxazolidin-5-ylmethoxy]-5-(10-hydroxydecyl)benzonitrile
- 2-[(2RS,5S)-3-tert-butyl-2-phenyloxazolidin-5-ylmethoxy]-5-(10-hydroxydecyl)benzonitrile
- 2-[(2RS,5S)-3-tert-butyl-2-phenyloxazolidin-5-ylmethoxy]-5-(3-hydroxypropyl)benzonitrile
- 4-[(2RS,5S)-3-tert-butyl-2-phenyloxazolidin-5-ylmethoxy]-7-(10-hydroxydecyl)-1H-indol
- 4-[(2RS,5S)-3-tert-butyl-2-phenyloxazolidin-5-ylmethoxy]-2-cyano-7-(3-hydroxypropyl)-1H-indol
- 4-[(2RS,5S)-3-tert-butyl-2-phenyloxazolidin-5-ylmethoxy]-1-(16-hydroxyhexadecyl)benzol
- 2-[(2RS,5S)-3-tert-butyl-2-phenyloxazolidin-5-ylmethoxy]-5-(2-hydroxyethyl)-benzonitrile
- 5-hydroxy-2-[(2RS,5S)-3-tert-butyl-2-phenyloxazolidin-5-ylmethoxy]benzonitrile
- 4-[(2RS,5S)-3-tert-butyl-2-phenyloxazolidin-5-ylmethoxy]-1H-indol-6-carboxylic acid-(8-hydroxyoctyl)amide.

10. Use of a compound of claim 1 as a pharmaceutical.

CLAIMS: (ART. 167(2)(a))

1. A process for the preparation of a - - - - 4-aryl-1,4-dihydropyridine-3- and/or 5-carboxylic acid este derivative wherein at least one ester group is additionally substituted by a β-blocker moiety

with the proviso that it is other than a compound of formula Y

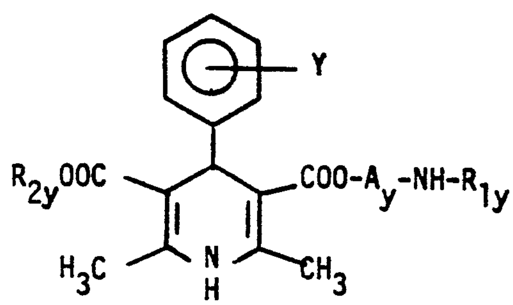

Y

wherein

either

Y is nitro,

$R_{1y}$ is 2-hydroxy-2-phenylethyl or 2-hydroxy-3-phenoxypropyl,

$R_{2y}$ is alkyl of 1 to 10 carbon atoms and

$A_y$ is alkylene of 1 to 10 carbon atoms optionally interrupted by an oxygen atom,

or

Y is hydrogen, nitro or trifluoromethyl,

$R_{1y}$ is 2-hydroxy-3-phenoxypropyl optionally substituted in the phenyl ring by halogen, lower alkyl, allyl, loweralkoxy (lower)-alkyl, allyloxy, cyano or ethinyl, or is 2-hydroxy-3-indolyl-oxypropyl,

$R_{2y}$ is lower alkyl and

$A_y$ is $-CH_2CR^yR^{y'}-$ wherein $R^y$ and $R^{y'}$ independently are hydrogen or methyl,

in free form or where appropriate in salt form.

which comprises condensing a corresponding 4-aryl-1,4-dihydropyridine-3- and/or 5-carboxylic acid ester derivative having an appropriate reactive group in place of the β-blocker moiety, or a precursor thereof, with a corresponding, reactive β-blocker, or a precursor thereof.

and recovering the resultant compound in free form or where appropriate in salt form.

2. A process according to claim 1 for the preparation of a ------ compound of formula I

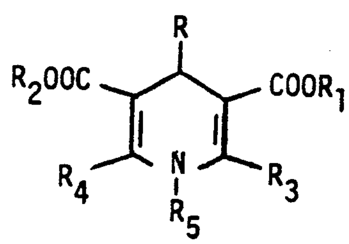

I

wherein

R is optionally substituted aryl,

$R_1$ is a group usual in the 4-aryl-1,4-dihydropyridine-3,5-dicarboxylic acid ester field but additionally substituted by a β-blocker moiety,

$R_2$ is a group usual in the 4-aryl-1,4-dihydropyridine-3,5-dicarboxylic acid ester field or independently has the significance indicated above for $R_1$ and

$R_3$, $R_4$ and $R_5$ independently are a group usual in the 4-aryl-1,4-dihydropyridine-3,5-dicarboxylic acid ester field,

with the proviso that it is other than a compound of formula Y as defined in claim 1,

in free form or where appropriate in salt form.

3700 /VA